**Europäisches Patentamt**

**European Patent Office**

**Office européen des brevets**

(19)

(11) Veröffentlichungsnummer: **0 026 476**
**B1**

# EUROPÄISCHE PATENTSCHRIFT

(12)

(45) Veröffentlichungstag der Patentschrift:
**12.09.84**

(21) Anmeldenummer: **80105826.4**

(22) Anmeldetag: **25.09.80**

(51) Int. Cl.³: **A 61 N 1/36,** H 03 K 5/00

(54) **Herzschrittmacher.**

(30) Priorität: **27.09.79 DE 2939254**

(43) Veröffentlichungstag der Anmeldung:
**08.04.81 Patentblatt 81/14**

(45) Bekanntmachung des Hinweises auf die Patenterteilung:
**12.09.84 Patentblatt 84/37**

(84) Benannte Vertragsstaaten:
**CH DE FR GB IT LI NL SE**

(56) Entgegenhaltungen:
**DE - A - 2 006 076**
**DE - A - 2 151 426**
**DE - A - 2 236 434**
**DE - A - 2 452 516**
**DE - A - 2 755 757**

**SIEMENS DATENBUCH 1974/75, Band 1, "Digitale Schaltungen MOS", Siemens AG", Seiten 217-219**

(73) Patentinhaber: **SIEMENS AKTIENGESELLSCHAFT, Berlin und München Wittelsbacherplatz 2, D-8000 München 2 (DE)**

(72) Erfinder: **Elmqvist, Häkan, Dr., Sunnerdahlsvägen 7, S-161 38 Bromma (SE)**

# Beschreibung

Die Erfindung bezieht sich auf einen Herzschrittmacher mit einem Impulsgeber für Herzschrittmacher-Impulse, der durch einen primären Frequenzgeber mit vorgebbarer Primärfrequenz steuerbar ist, und mit einer einen Einstellgeber aufweisenden Steuerschaltung zur Einstellung erwünschter Pulsfolgefrequenzen des Impulsgebers für die Herzschrittmacher-Impulse in Abhängigkeit vom Einstellwert des Einstellgebers.

Ein Herzschrittmacher dieser Art ist beispielsweise durch die DE-A-2 006 076 vorbekannt. Eigenart dieses Herzschrittmachers ist es jedoch, dass die Vorwahl erwünschter Impulsfolgefrequenzen nur durch Einstellung des Zeitintervallwertes zwischen zwei aufeinanderfolgenden Impulsen in Millisekunden möglich ist. Die Einstellung nach Impulsintervallen ist jedoch eine im allgemeinen unübliche Form der Herzfrequenzvorwahl. Die Herzfrequenz ist nicht nur für die Allgemeinheit, sondern im speziellen Fall auch für den Fachmann, d.h. Arzt, das geläufigere Mass zur Beurteilung der Herztätigkeit eines Patienten. Aus der Angabe eines Herzfrequenzwertes lässt sich dann im allgemeinen sofort ermitteln, ob ein für den Patienten kritischer Zustand der Herzaktivität vorliegt oder nicht. Entsprechend kann auch im Falle von Herzschrittmacher-Patienten der Herzschrittmacher in seiner Ausgangsfrequenz nur dann optimal an die Bedürfnisse des Patienten angepasst werden, wenn die Frequenz, auf die sich der Herzschrittmacher einstellen soll, von vornherein in Impuls/min eingestellt wird.

Aus der DE-A-2 755 757 ist ein Herzschrittmacher bekannt, bei dem in der Steuerschaltung das primäre Frequenzsignal eines primären Frequenzgebers einem einstellbaren Binär-Frequenzteiler zuführbar ist.

Aufgabe der vorliegenden Erfindung ist es, bei einem Herzschrittmacher der eingangs genannten Art eine solche Einstellung Impuls/min vorzusehen, wobei jedoch der technische Aufwand insgesamt optimal gering gehalten werden soll.

Die Aufgabe wird erfindungsgemäss dadurch gelöst, dass in der Steuerschaltung das primäre Frequenzsignal des primären Frequenzgebers sowie ein in Abhängigkeit von einem Puls/min-Einstellwert eines Puls/min-Einstellgebers einstellbares binäres Multiplikationssignal eines Multiplikationssignalgebers, einem Binär-Frequenz-Multiplikator zuleitbar sind, der die primäre Frequenz $f_0$ des primären Frequenzsignals auf eine sekundäre Frequenz wandelt, die sich aus dem durch den Maximalwert n max des Multiplikationssignals vorgegebenen Bruchteil: $f_0/(n_{max}+1)$ der primären Frequenz multipliziert mit dem dem Einstellwert entsprechenden Binärwert n des Multiplikationssignals.

Die Erfindung erlaubt in einfachster Weise also die Einstellung in Abhängigkeit von einem Puls/min-Einstellwert. Der technische Aufwand zur Erzielung dieses Ergebnisses ist optimal gering. Binär-Frequenz-Multiplikatoren, wie sie zur Erzielung des erfindungsgemässen Ergebnisses u.a. eingesetzt werden, sind im Handel erhältlich und beispielsweise im DATABOOK RCA Solid State «COS/MOS Integrated circuits» aus dem Jahre 1978, z.B. auf der Seite 242 speziell für 4-Bit-Ausführung, beschrieben.

Weitere vorteilhafte Ausgestaltungen der Erfindung ergeben sich aus den Unteransprüchen.

Im nachfolgenden wird anhand zweier Figuren ein Ausführungsbeispiel der Erfindung näher erläutert.

Es zeigen:

Fig. 1 ein Ausführungsbeispiel der Erfindung im Prinzipschaltbild,

Fig. 2 einen Erweiterungszusatz im Prinzipschaltbild der Fig. 1.

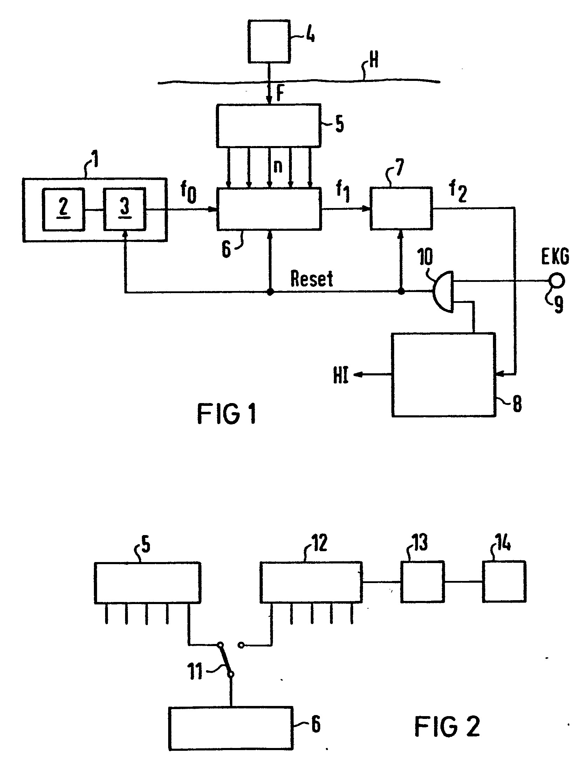

Die Fig. 1 zeigt unter der Kennziffer 1 einen primären Frequenzgeber, der Ausgangsimpulse der Pulsfolgefrequenz $f_0$ erzeugt. Beispielsweise soll im vorliegenden Fall die Frequenz $f_0$ = 333,3 Hz betragen. Der primäre Frequenzgeber 1 umfasst z.B. einen Frequenzoszillator 2, der auf einer bestimmten Frequenz schwingt, die durch einen nachfolgenden Frequenzuntersetzer 3, z.B. Zähler, auf die erwünschte Frequenz $f_0$ heruntergesetzt wird. Die erwähnte primäre Frequenz $f_0$ wird nun zusammen mit einem binären ganzzahligen Multiplikationssignal n eines Multiplikationssignalgebers 5, das in Abhängigkeit von einem Puls/min-Einstellwert F eines Puls/min-Einstellgebers 4 einstellbar ist, einem Binär-Frequenz-Multiplikator 6 zugeleitet. Der Binär-Frequenz-Multiplikator wandelt die primäre Frequenz $f_0$ auf eine sekundäre Frequenz $f_1$, die sich ergibt aus dem durch den Maximalwert $n_{max}$ des Multiplikationssignals vorgegebenen Bruchteil $f_0/(n_{max}+1)$ der primären Frequenz multipliziert mit dem dem Einstellwert entsprechenden Binärwert n des Multiplikationssignals. Im vorliegenden Fall beträgt also, ausgehend von einer Frequenz $f_0$ = 333,3 Hz, die Frequenz $f_1$ bei Anwendung eines 5-Bit-Binär-Frequenz-Multiplikators mit $n_{max}$ = 31, also $n_{max}+1$ = 32, $f_1$ = n × 10,42 Hz.

Die Ausgangsfrequenz n × 10,42 Hz ist allerdings zur Vorgabe einer Reizimpulsfolgefrequenz im Herzfrequenzbereich immer noch um etwa einen Faktor 100 zu gross. Aus diesem Grunde folgt speziell im vorliegenden Ausführungsbeispiel auf den Binär-Frequenz-Multiplikator 6 ein Frequenzuntersetzer 7 (Zähler), der die noch zu hohe Ausgangsfrequenz $f_1$ des Binär-Frequenz-Multiplikators 6 durch den Faktor 125 teilt. Das Ergebnis ist eine Ausgangsfrequenz des Frequenzuntersetzers 7 von $f_2$ = n × 5 Impulse/min. Damit ergibt sich also mit dem Ausführungsbeispiel die Einstellmöglichkeit in 5 Impuls/min-Schritten bei n = 0 bis n = $n_{max}$ = 31 im Bereich $f_1$ = 0 bis 155 Impuls/min. Interessant ist für den Anwendungsfall am Patienten allerdings meistens nur der Bereich von etwa 30 bis 150 Impuls/min. Dieser Bereich lässt sich dann mit Einstellung von Werten zwischen n = 6 bis 30 erreichen.

Im vorliegenden Ausführungsbeispiel haben die verwendeten Frequenzen und Bauelemente lediglich exemplarischen Charakter. Selbstverständlich könnte auf zusätzliche Untersetzerglieder immer dann verzichtet werden, wenn von vornherein sehr kleine primäre Frequenzen $f_0$ verwendet werden. Diese Frequenzen müssten dann allerdings im Bereich 3 Hz und darunter liegen. Dies führt jedoch u. U. zu unerwünschten Unregelmässigkeiten in der Ausgangsfrequenz des Binär-Frequenz-Multiplikators. Weitere Variationsmöglichkeiten ergeben sich dadurch, dass Binär-Frequenz-Multiplikatoren 6 mit höheren Verarbeitungs-Bit-Zahlen als beispielsweise 5-Bits eingesetzt werden. Mit steigender Bit-Zahl erhöht sich entsprechend $n_{max}$ und damit der Teilungsfaktor für die primäre Frequenz $f_0$. Beträgt im vorliegenden Falle bei Verwendung eines 5-Bit-Binär-Frequenz-Multiplikators der Faktor $n_{max}$ noch 31, so beträgt er beispielsweise im Falle der Anwendung eines 7-Bit-Binär-Frequenz-Multiplikators bereits 255. Im Zusammenspiel mit einer entsprechend gewählten primären Eingangsfrequenz $f_0$ ergeben sich hiermit ebenfalls andere Teilungswerte für die Ausgangsfrequenz. Im Ausführungsbeispiel der Fig. 1 ist ferner eine Frequenzeinstellung in 5 Impuls/min-Schritten vorgesehen. Selbstverständlich können durch entsprechende Modifikationen auch kleinere Teilschrittwerte, z.B. im Bereich 1 Impuls/min oder 2 Impuls/min oder sonstiger Zwischenwerte, vorgewählt werden.

Gemäss Darstellung der Fig. 1 wird im Ausführungsbeispiel die Frequenz $f_2 = n \times 5$ Impulse/min einer Endstufe 8 zugeführt, die im vorliegenden Falle den Impulsgeber für die Herzschrittmacher-Impulse darstellt. Die Herzschrittmacher-Impulse sind mit HI bezeichnet; sie werden über den dargestellten Ausgang des Impulsgebers 8 in Pfeilrichtung über einen Elektrodenanschluss auf das Herz des Schrittmacher-Patienten gegeben. Das Ausführungsbeispiel der Fig. 1 zeigt ferner einen inhibierten Schrittmacher. Dieser weist also einen Eingang 9 auf, an dem durch Überwachung des EKG des Patienten (z.B. auch durch Abgriff über die Herzschrittmacher-Elektrode) spontane Herzreaktionen erfasst und auf den einen Eingang eines UND-Gliedes 10 gegeben werden. Der zweite Eingang des UND-Gliedes liegt am Impulsgeber 8 für die Herzschrittmacher-Impulse. Tritt am Eingang 9 demnach ein spontaner Herzimpuls auf, so wird über das UND-Glied 10 ein Resetimpuls für die Kettenschaltung aus primärem Frequenzgeber 1, Binär-Frequenz-Multiplikator 6 und Frequenzuntersetzer 7 gegeben, durch den sämtliche angesteuerte Elemente dieser Kette auf den Ausgangszustand gesetzt werden. Dies geschieht so oft, wie über den Eingang 9 weitere spontane Herzaktionen registriert werden.

Im Ausführungsbeispiel der Fig. 1 ist der Puls/min-Geber 4 ein vom eigentlichen Schrittmacher separates Gerät. Er wird also im vorliegenden Falle nicht zusammen mit dem Schrittmacher implantiert; vielmehr ist der Puls/min-Geber 4 dahingehend ausgebildet, dass er transkutan, d.h. durch die Hautoberfläche H hindurch, die Einstellsignale zum implantierten Multiplikationssignalgeber 5 übermittelt. Der Multiplikations-Signalgeber 5 ist zur Einspeicherung jeweils vom Puls/min-Geber 4 übermittelter Informationen F als Programmspeicher ausgebildet. Eine einmal gegebene Information wird demnach so lange beibehalten, bis sie durch Eingabe neuer Information von aussen durch den Puls/min-Geber 5 ersetzt wird.

In der Fig. 2 ist noch eine Modifikation des Ausführungsbeispiels der Fig. 1 dargestellt. Die Abänderung besteht darin, dass in der Anordnung dem Multiplikations-Signalgeber 5 ausgangsseitig ein Umschalter 11 zugeordnet ist, der es erlaubt, den Binär-Frequenz-Multiplikator 6 eingangsseitig vom Ausgang des Multiplikations-Signalgebers 5 auf den Ausgang eines Testfrequenzgebers 12, 13 zur Abgabe einer Testfrequenz zum Testen insbesondere des Zustandes der Batterie 14 des Herzschrittmachers umzuschalten. Der Testfrequenzgeber umfasst in der prinzipiellen Darstellung einen zweiten Multiplikations-Signalgeber 12 für die Abgabe solcher Binär-Werte n für den Binär-Frequenz-Multiplikator 6, die an die Testfrequenz entsprechend angepasst sind. Das Bauteil 13 ist ein Analog-Digital-Wandler für die eingestellte Testfrequenz.

Die anhand des Ausführungsbeispiels erläuterte Erfindung ermöglicht also bei technisch einfachstem Aufbau die Vorwahl der Herzschrittmacher-Frequenz durch Direkteinstellung in Impulsen/min. Im Gegensatz zum Stand der Technik, wo lediglich eine Voreinstellung in Millisekunden (ms) entsprechend dem Folgeintervall T der Herzschrittmacher-Impulse möglich ist, ergibt sich somit eine Einstellmöglichkeit, die jetzt besser als bisher an die Gewohnheit angepasst ist. Eine Einstellung kann aus diesem Grunde jetzt sehr viel einfacher und sicherer erfolgen.

**Patentansprüche**

1. Herzschrittmacher, mit einem Impulsgeber (8) für Herzschrittmacher-Impulse (HI), der durch einen primären Frequenzgeber (1) mit vorgebbarer Primärfrequenz ($f_0$) steuerbar ist, und mit einer einen Einstellgeber (4) aufweisenden Steuerschaltung zur Einstellung erwünschter Pulsfolgefrequenzen des Impulsgebers (8) für die Herzschrittmacher-Impulse in Abhängigkeit vom Einstellwert des Einstellgebers, dadurch gekennzeichnet, dass in der Steuerschaltung das primäre Frequenzsignal ($f_0$) des primären Frequenzgebers (1) sowie ein in Abhängigkeit von einem Puls/min-Einstellwert (F) eines Puls/min-Einstellgebers (4) einstellbares binäres Multiplikationssignal (n) eines Multiplikationssignalgebers (5) einem Binär-Frequenz-Multiplikator (6) zuleitbar sind, der die primäre Frequenz $f_0$ des primären Frequenzsignals auf eine sekundäre Frequenz ($f_1$) wandelt, die sich ergibt aus dem durch den Maximalwert $n_{max}$ des Multiplikationssignals vorgegebenen Bruchteil: $f_0/(n_{max} + 1)$ der primären Frequenz multipliziert mit dem dem Einstellwert entsprechenden Binärwert n des Multiplikationssignals.

2. Herzschrittmacher nach Anspruch 1, dadurch gekennzeichnet, dass der Puls/min-Geber in vorgebbaren Puls/min-Schritten (z.B. in 1 oder 5 oder 10 Puls/min-Schritten) einstellbar ist und dass der Multiplikationssignalgeber ein Programmspeicher (5) ist, der signalmässig mit dem Puls/min-Geber (4) derart koppelbar ist, dass in Abhängigkeit von der eingestellten Schrittzahl am Programmspeicher selbsttätig das dem eingestellten Schritt zugehörige ganzzahlige binäre Multiplikationssignal (n) eingestellt wird.

3. Herzschrittmacher nach Anspruch 1 oder 2, dadurch gekennzeichnet, dass zur bedarfsmässigen weiteren Intervallunterteilung von Puls/min-Schritten dem Binär-Frequenz-Multiplikator (6) Frequenzuntersetzer (z. B. 7) vor- und/oder nachgeschaltet, sind.

4. Herzschrittmacher nach einem der Ansprüche 1 bis 3, dadurch gekennzeichnet, dass der Programmspeicher (5) Bestandteil des zu implantierenden Schrittmachers ist und dass der Puls/min-Geber (4) zur transkutanen Übermittlung seiner Einstellsignale zum Programmspeicher ausgebildet ist.

5. Herzschrittmacher nach einem der Ansprüche 1 bis 4, dadurch gekennzeichnet, dass bei Ausbildung als inhibierter oder synchroner Schrittmacher ein Rücksetzeingang für den Binär-Frequenz-Multiplikator (6) sowie gegebenenfalls für weitere Schaltungsteile (3, 7) vorhanden ist, der bei Auftreten von spontanen Herzaktionen den Binär-Frequenz-Multiplikator sowie gegebenenfalls die anderen Schaltungsteile auf einen vorgewählten Initialzustand setzt.

6. Herzschrittmacher nach. einem der Ansprüche 1 bis 5, gekennzeichnet durch einen Umschalter (11) zur Umschaltung des Binär-Frequenz-Multiplikators (6) eingangsseitig von Ausgangssignalen des Einstellgebers (5) auf Ausgangssignale für eine Testfrequenz eines Testfrequenzgebers (12, 13) zum Testen des Batteriezustandes (14).

**Claims**

1. Heart pacemaker comprising a pulse generator (8) for heart pacemaker pulses (HI) which is controllable by means of a primary frequency generator (1) having a predeterminable primary frequency $(f_0)$, and comprising a control circuit which has a setting indicator (4) and serves to set required pulse train frequencies of the pulse generator (8) for the heart pacemaker pulses in dependence upon the setting value of the setting indicator, characterised in that in the control circuit, the primary frequency signal $(f_0)$ of the primary frequency generator (1) and a binary multiplication signal (n) of a multiplication signal generator (5) which can be set in dependence upon a pulse/min setting value (F) of a pulse/min setting indicator (4), can be fed to a binary frequency multiplier (6) which changes the primary frequency $(f_0)$ of the primary frequency signal into a secondary frequency $(f_1)$ which results from the fraction: $f_0/(n_{max}+1)$, which is predetermined by the maximum value $n_{max}$ of the multiplication signal, of the primary frequency multiplied by the binary value n of the multiplication signal which corresponds to the setting value.

2. Heart pacemaker as claimed in claim 1, characterised in that the pulse/min indicator can be set in predeterminable steps (e.g. in 1 or 5 or 10 pulse/min steps) and that the multiplication signal generator is a programme store (5) which can be coupled to the pulse/min indicator (4) signal-wise in such a manner that in dependence upon the set number of steps at the programme store, the integral binary multiplication signal (n), which is assigned to the set step, is automatically set.

3. Heart pacemaker as claimed in claim 1 or 2, characterised in that for the possible further interval division of pulse/min steps, frequency dividers (e.g. 7) are connected preceding and/or following the binary frequency multiplier (6).

4. Heart pacemaker as claimed in one of claims 1 to 3, characterised in that the programme store (5) is a component of the pacemaker to be implanted, and that the pulse/min indicator (4) is designed for the transcutaneous transmission of its setting signals to the programme store.

5. Heart pacemaker as claimed in one of claims 1 to 4, characterised in that in the design as an inhibited or synchronous pacemaker a resetting input is present for the binary frequency multiplier (6) and possibly for further circuit components (3, 7), which resetting input brings the binary frequency multiplier and possibly the other circuit component into a preselected initial state when spontaneous heart actions occur.

6. Heart pacemaker as claimed in one of claims 1 to 5, characterised by a change-over switch (11) for changing over the binary frequency multiplier (6) at the input end from output signals of the setting indicator (5) to output signals for a test frequency of a test frequency generator (12, 13) in order to test the battery state (14).

**Revendications**

1. Stimulateur cardiaque comportant un générateur d'impulsions (8) pour les impulsions du stimulateur cardiaque (HI), lequel générateur est susceptible d'être commandé avec une fréquence primaire $(f_0)$ par un générateur de fréquence primaire (1), ainsi qu'un générateur de réglage (4) pourvu d'un circuit de commande pour régler la fréquence de répétition des impulsions du générateur d'impulsions (8) par les impulsions du stimulateur cardiaque en fonction de la valeur de réglage du générateur de réglage, caractérisé par le fait que dans le circuit de commande, le signal de fréquence primaire $(f_0)$ du générateur de fréquence primaire (1), de même qu'un signal binaire de multiplication (n) d'un générateur de signaux de multiplication (5) et réglable en fonction d'une valeur de réglage impulsions/minute (F) d'un générateur de réglage d'impulsions/minute (4), sont susceptibles d'être appliqués à un multiplicateur de fréquence binaire (6) qui convertit la fréquence primaire $(f_0)$ du signal de fréquence primaire en une fréquence secondaire $(f_1)$ qui résulte de la fraction: $f_0/(n_{max}+1)$ prédéterminée par

la valeur maxima $n_{max}$ du signal de multiplication, de la fréquence primaire multipliée par la valeur binaire (n) du signal de multiplication, qui correspond à la valeur de réglage correspondante.

2. Stimulateur cardiaque selon la revendication 1, caractérisé par le fait que le générateur d'impulsions/minute est réglable en pas d'impulsions/minute donnés à l'avance, (par exemple en pas de 1, ou 5 ou 10 impulsions/minute) et que le générateur de signaux de multiplication est une mémoire de programme (5) qui est capable d'être couplée, du point de vue des signaux, au générateur d'impulsions/minute (4) de façon qu'en fonction du nombre de pas réglés au niveau de la mémoire de programme, le nombre de signaux de multiplications binaires et entier associé au pas de réglage, se règle automatiquement.

3. Stimulateur cardiaque selon la revendication 1 ou 2, caractérisé par le fait que pour la subdivision supplémentaire des pas d'impulsions/minute dont on aurait besoin, on monte en amont et/ou en aval du multiplicateur binaire de la fréquence (6) un démultiplicateur de fréquence (par exemple 7).

4. Stimulateur cardiaque selon l'une des revendications 1 à 3, caractérisé par le fait que la mémoire de programme (5) est un élément constitutif du stimulateur cardiaque à implanter et que le générateur d'impulsions/minute (4) est réalisé pour une transmission transcutanée de ces signaux de réglage à la mémoire de programme.

5. Stimulateur cardiaque selon l'une des revendications 1 à 4, caractérisé par le fait que dans le cas de la réalisation sous la forme d'un stimulateur cardiaque inhibé ou synchrone, il est prévu une entrée de remise à l'état initial pour le multiplicateur de fréquence binaire (6) ainsi qu'éventuellement pour d'autres éléments de circuits (3, 7), laquelle entrée de remise à l'état initial place le multiplicateur de fréquence binaire de même qu'éventuellement les autres éléments du circuit à un état initial prédéterminé, lors de l'apparition de réactions cardiaques spontanées.

6. Stimulateur cardiaque selon l'une des revendications 1 à 5, caractérisé par un commutateur pour commuter, du côté entrée, le multiplicateur de fréquences binaires, de signaux de sortie du générateur de réglage (5) à des signaux de sortie pour une fréquence de test d'un générateur de fréquence de test (12, 13) pour tester l'état de la batterie (14).

**FIG 1**

**FIG 2**